# EUROPEAN PATENT SPECIFICATION

(11) **EP 2 618 042 B1**
(45) Date of publication and mention of the grant of the patent: **13.01.2016**
(21) Application number: 13151548.8
(22) Date of filing: 17.01.2013
(51) Int. Cl.: F21S 2/00, F21V 21/34, A61B 19/00, F21V 14/02, F21V 21/15, F21W 131/205, F21Y 101/02

(54) **A better lighting arrangement of an operating theatre**
Vererbesserte Beleuchtungsanordnung für einenOperationssaal
Dispositif d'éclairage d'une salle d'opération

(30) Priority: 17.01.2012 FI 20125054
(43) Date of publication of application: 24.07.2013
(73) Proprietor: Merivaara Oy, 15150 Lahti (FI)
(72) Inventor: Bärlund, Paul, FI-15170 Lahti (FI)
(74) Representative: Berggren Oy Ab

(56) References cited:
- US-A- 5 347 431
- US-A1- 2006 067 089

## Description

The invention relates to an improved lighting arrangement as set forth in the preamble of claim 1, particularly for an operating theatre or the like facility.

Operating theatre requires high quality lighting, especially for those parts of operating theatre where surgeries are performed. In addition, lighting is concentrated specifically on a part of the patient's body to be treated with surgical procedures.

The conventional operating theatre lighting arrangement comprises a general lighting arrangement and one or more surgical light fixtures set up in the proximity of and above the operating table. The prior known surgical light fixture comprises a fixed light fixture carrier, which is provided with a plurality of light sources such as halogen lamps or the like, and a generally pivotable spring arm for attaching the light fixture to ceiling structures and for adjusting its position relative to the operating table and the surgical patient resting thereon.

Prior art known from German patent publication DE 102006040393 is a surgical lighting fixture, which comprises a plurality of lighting segments assembled on a single carrier and implemented with light emitting diodes or LEDs equipped with optics. This surgical lighting fixture is also intended for attachment by way of a spring arm to the ceiling structures of an operating theatre.

The prior known surgical lights are generally quite similar in design. A problem with surgical lights is particularly a carrier structure and the attachment to ceiling structures. The light carrier is fixed, heavy and clumsy. The adjustment of a lighting fixture position takes place manually with a hazard of the lighting fixture colliding with other operating theatre equipment during its relocation. Another problem during a procedure results from the surgeon's possible contacts with non-sterile surfaces. Cleaning the lighting fixture is also a tedious process.

Yet another problem with prior known operating theatre lights is that most of the time such fixtures are installed in an operating theatre within the supply area of clean incoming air. It is an object of the incoming airflow to deny the access of contaminants onto the operating table and to the patient's surgical site, but the equipment, especially surgical light fixtures interfering with the airflow, muddles up the downward directed laminar airflow and thereby jeopardizes patient safety. An object of the invention, among other things, is to eliminate problems related to prior known surgical lights. It is also an object of the invention to provide a new improved operating theatre lighting arrangement, which preferably enables the management of even the general lighting of an operating theatre, and particularly task lighting for an operating table and thereby for surgical procedures.

The lighting arrangement according to the invention is characterized by what is presented in claim 1. Preferred embodiments of the invention are presented in dependent claims.

The invention relates to a lighting arrangement which is implemented by means of light emitting diodes or LEDs. The lighting arrangement comprises a lighthead and an incoming air frame, which lighthead is constituted by a frame fitted with a plurality of elongated rod type mounting supports, said mounting supports being provided with a plurality of light elements containing at least one light emitting diode. The mounting supports are organized in two groups, each of said groups including at least two mounting supports, whereof the first group is set on a first level (A-A) and the second group on a second level (B-B), such that the mounting supports of each group are apart or spaced by a gap from each other, and that the first group's mounting supports lie at an angle, most preferably at a right angle, relative to the second group's mounting supports, and the lighthead, along with its frame and mounting supports, is set up in the proximity of a ceiling (K) in the operating theatre or the like facility substantially above an operating table (LP), such that the location thereof is below the illuminated facility's incoming air frame (TF).

In a preferred embodiment of the invention, the frame has a quadrangular shape, such as rectangular or square, said frame including first and second frame members, which are pairwise parallel.

In a second preferred embodiment of the invention, the lighting arrangement has its mounting supports attached to the frame in a movable manner, such that individual mounting supports are movable relative to each other. Hence, clearances between the mounting supports are adjustable, yet most preferably in such a way that there is always a small gap, a minimum gap, between the mounting supports. In this case, the mounting supports are movable along guides, such that the air resistance thereof remains constant in a direction transverse, preferably perpendicular, relative to mounting levels established by the mounting supports.

Thus, the mounting supports are preferably maneuvered along the guides in such a way that the lighthead's surface area, which is penetrated by an airflow arriving at the lighthead from the incoming air frame, remains substantially unchanged in a direction transverse, preferably perpendicular, relative to the mounting levels. In practice, this implies that the mounting supports are manipulated in such a way that, in a direction transverse relative to the mounting levels, the direction of a mounting support remains constant with respect to an airflow arriving at the lighthead from the incoming air frame (TF).

In a third preferred embodiment of the invention, the ends of each mounting support are fitted in the frame's guides, along which the mounting support is maneuverable with appropriate transfer means.

In a preferred embodiment of the invention, the mounting supports are substantially straight rigid and dimensionally stable shafts or rods, which are preferably also channel-shaped. These are jointly or in each group most preferably set in a parallel relationship at a distance or spaced from each other to extend across an area that requires a particularly high light intensity.

In a preferred embodiment of the invention, the light elements are fitted on a mounting support successively in a row at a distance from each other.

In a preferred embodiment of the invention, at least some of the light elements comprise not only a light source, i.e. one or more light emitting diodes, but also adjustable optics for focusing the light emitted by the light source, and alignment elements, such as one or more reversible motors, for directing the light, especially a light beam, emitted by the light source.

Patent document CN101769482 relates to a lamp cap of a shadowless lamp which is said to own a shall air resistant coefficient and a little effect on clean air stream. This lamp is suitable for operating rooms. However in patent document CN101769482 the mounting supports lie on the same plane. In the present invention there are two groups of mounting supports and each group are apart or spaced by a gap from each other and the first group's mounting supports lie at an angle, most preferably at a right angle, relative to the second group's mounting supports.

It is a benefit of the invention that a light field produced by the lighting arrangement can be arbitrarily manipulated. Focusing the illumination on the operating table area, and particularly on the patient's surgical site, is implementable with a lighting arrangement of the invention. The focused light field is manipulable in terms of its extent and shape according to surgical requirements and the situation.

It is a benefit of the invention that the general lighting of an operating theatre is also implementable with a lighting arrangement of the invention.

It is a benefit of the invention that the risk of physical contact with the lighting arrangement's light sources, or with its components in general, is practically eliminated, and the possible access of germs to the surgical incision by way of contact with the lighting arrangement within a sterile area in the vicinity of an operating table is prevented. With respect to floor level, the lighting arrangement is generally located at a height beyond a reach of the surgeon or any other assisting person working at the operating table. Therefore, the infection risk after a surgical procedure is lower than before. It should also be noted that the assisting staff need not enter the sterile area in order to adjust a surgical light, because the surgical light is integrated in the lighting arrangement set in the proximity of an operating theatre ceiling. Another benefit of the invention is that the movable and/or pivotable equipment and/or accessories of an operating theatre do not collide with the lighting arrangement's light sources or its components in general.

It is a benefit of the invention that the sterile incoming airflow, which is supplied in from the ceiling structures of an operating theatre and directed towards the floor, can be kept laminar. The mounting supports included in the lighting arrangement are aerodynamic in the above-discussed flow direction, thereby neither blocking the passage of air nor deflecting significantly the advancing direction of air. The sterile airflow coming towards an operating table from above finds its way without interference to the surgical area and contributes effectively to blocking the access of germs to the surgical incision. Therefore, the infection risk after a surgical procedure is also in this respect lower than before.

In a lighting arrangement of the invention, it is a benefit that the permanent components of a lighthead, such as the mounting supports, have a surface area which is small on the lighthead's level in comparison with the lightheads of available lighting arrangements and with respect to the attachment arms thereof. It is by virtue of the small surface area of the lighthead's permanent components that air is able to flow through the lighthead as opposed to prior known light fixtures, especially surgical light fixtures.

A benefit in a lighting arrangement of the invention is a simple attachment of the lighthead to ceiling structures. The lighthead is suspended by its various sides with suitable suspension elements, such as with a plurality of suspension ropes or the like suspenders, from ceiling structures, especially outside an area of the operating theatre defined by the incoming air frame. Hence, there is no need for any ceiling fastener located in a middle ceiling area and inside the incoming air frame. The lighthead suspension arrangement also relieves and simplifies the ceiling structure.

A benefit of the invention is that the lighting arrangement is simple to provide with suitable accessories, such as a camera arrangement and light focusing elements.

A benefit offered by a lighting arrangement of the invention is versatility and programmability, which enable various lighting functions to be performed with one and the same physical assembly.

A benefit offered by a lighting arrangement of the invention is a simple construction and manufacturing friendliness. Another benefit of the invention is good usability, reliability and safety.

Still another benefit offered by a lighting arrangement of the invention is that it is almost maintenance-free, because, i.a., the light elements equipped with LEDs have a long service life.

The invention and its other benefits will now be described in more detail with reference to the accompanying drawing, in which
- fig. 1: shows in a perspective view an operating theatre, especially an operating theatre ceiling, on which is mounted a lighting arrangement of the invention;
- fig. 2: shows in a partially sectional side view the lighting arrangement shown in fig. 1;
- fig. 3: shows illustratively in a partially cross-sectional view a light element fitted on a mounting support; and
- fig. 4: shows schematically a lighting arrangement control unit and its connection to the lighting arrangement's light elements and to a remote controller.

In the figures, like components are designated with like reference numerals.

The invention relates to a lighting arrangement, which is implemented by means of light emitting diodes or LEDs.

The lighting arrangement according to the invention is intended particularly for an operating theatre or the like facility, yet it is also adaptable to other types of rooms or facilities, such as workrooms, which require powerful, preferably focusable illumination, i.a. for carrying out a high precision and demanding work assignment.

The lighting arrangement comprises a lighthead 1, which is established by a frame 5 fitted with a plurality of elongated rod type mounting supports 2; 21, 22; 21¹, 21²,..., 21ⁿ; 22¹, 22² ..., 22ⁿ (n = positive integer) spaced by a gap 4; 4a, 4b from each other. The mounting supports 2 are provided with a plurality of light elements 3, comprising at least one light emitting diode 31 to function as a light source.

In the invention, the mounting supports 2 are organized in two groups 21, 22, each of said groups including at least two mounting supports 21; 21¹, 21²; 22; 22¹, 22². The first group 21 is set on a first level A-A and the second group 22 respectively on a second level B-B. The groups have their mounting supports 21; 21¹, 21²; 22; 22¹, 22² spaced, i.e. with the gap 4; 4a, 4b from each other. In addition, the first group has its mounting supports 21; 21¹, 21² at an angle, preferably at a right angle, to those of the second group 22; 22¹, 22². The first and second groups have their mounting supports 21, 22, and thereby their levels A-A and B-B, at a small distance, such as 25-50 mm, from each other. In a most preferred case, the first and second groups' mounting supports 21, 22 are vertically in alignment. The gaps 4; 4a, 4b between the mounting supports 2; 21, 22 in each group are unobstructed.

At least in a direction C-C, which is transverse, most preferably perpendicular, relative to a mounting level established by the mounting supports, i.e. relative to the first level A-A and the second level B-B, the mounting supports 2; 21, 22; 21¹, 21²; 21¹, 21² are aerodynamic rod type or shaft type elements, the air being readily able to flow through the gaps 4; 4a, 4b therebetween. Hence, the air is able to flow through the entire lighthead 1. Thus, the mounting supports produce a low air resistance in the airflow direction C-C, since the mounting supports have a sufficiently small surface area with respect to that of the gaps 4; 4a, 4b in the direction of the mounting levels A-A and B-B. The mounting supports 2 have a cross-section which is preferably a circle or ellipse, or at least the mounting supports 2 have a top portion 2a which is wedge-shaped and/or rounded. Thereby, the mounting supports 2 have a width I, which in the mounting level direction A-A or B-B falls preferably short of their height k.

As the lighthead 1 is placed below the incoming air frame TF and at the same time above the operating table LP, the airflow passing through the lighthead 1 arrives from the incoming air frame TF and proceeds through the mounting supports included in the lighthead's groups 21 and 22 in the direction C-C transverse to the respective mounting levels A-A and B-B, preferably in a perpendicular direction. The mounting supports have such a low air resistance in the direction of the mounting levels that the laminar airflow arriving from the incoming air frame TF passes through the lighthead in the direction C-C while retaining its laminarity.

The mounting support 2; 21, 22; 21¹, 21²; 21¹, 21² can have a length within the range of e.g. 150-300 cm, but other lengths are also possible depending on the intended use. The mounting support 2 has its width I for example within the bracket of 20-40 mm, but other width dimensions are also possible

The mounting supports 2; 21, 22; 21¹, 21²; 21¹, 21² are most preferably made of metal, such as aluminum or some other suchlike relatively lightweight, rigid and dimensionally stable material, such as appropriately reinforced plastic. The mounting supports 2 are rod type elements, which are preferably provided with a channel or a plurality of successive recesses for installing the light elements 3 therein.

In a preferred embodiment of the invention, the mounting supports 2; 21, 22; 21¹, 21²; 21¹, 21² are straight rods or shafts, which are disposed side by side at a distance from each other.

In a preferred embodiment of the invention, the light elements 3; 3¹, 3² are fitted on the mounting support 2; 21, 22; 21¹, 21²; 21¹, 21² in a row successively at a small distance from each other. The light elements 3 are disposed on one side of a plane, i.e. the mounting level, and in such a way that the light, especially light beams 311 produced by the light elements 3, is directed away from the level A-A (and B-B) to the other side thereof.

In one preferred embodiment of the invention, the width I of the mounting support 2; 21, 22; 21¹, 21²; 21¹, 21² in a plane perpendicular to its longitudinal direction, and at the same time on the mounting level A-A (and B-B), is preferably not more than what is a maximum diameter a of the light element 3 in its transverse plane. Alternatively, the maximum diameter of the light element 3, especially the diameter of its optics 32, may exceed the width of the mounting support.

In a preferred embodiment of the invention, at least some of the light elements 3; 3¹, 3², 3³ comprise not only an actual light source 31, i.e. a LED, but also adjustable optics 32, i.e. a suitable lens system and alignment elements 33, such as one or more electrically driven reversible motors, which are adapted to each other and most preferably fitted in a common housing 34. The light element 3 comprises preferably also a suitable transparent protective hood or at least a protective coating 35, which is provided as a protection for the optics 32 and through which propagates the light produced by a light source.

In one preferred embodiment of the invention, at least some of the light elements 3; 3¹, 3², 3³ are adapted to be movable in the mounting support 2; 21, 22; 21¹, 21²; 22¹, 22², especially in its longitudinal direction. In this case, the light element 3 is provided for example with a transfer pad, which is in turn adapted to be movable and displaceable in the mounting support 2, such as along the mounting support's lengthwise guide (a ridge or a groove), by a suitable power unit, such as an electric motor. In an alternative embodiment, the plurality of light elements 3; 3¹, 3², 3³ are accommodable on a single transfer pad, by means of which said elements can be maneuvered in the mounting support 2.

In one preferred embodiment of the invention, the light elements 3; 3¹, 3², 3³ have basic positions at a specific distance from each other, but are most preferably also movable lengthwise of the mounting support 2; 21, 22; 21¹, 21²; 22¹, 22².

The lighthead 1 has its frame 5 most preferably in a quadratic shape, such as rectangular or square. The frame 5 encircles or confines the mounting supports 2. The frame 5 comprises first and second sides. i.e. frame members 51, 52, 53, 54, which are straight sides. The first frame members 51, 52 of the frame 5 and, respectively, its second frame members 53, 54 are co-directional with each other and most preferably perpendicular to each other. The first frame members 51, 52 of the frame 5 are attached by their ends to the ends of the frame's second members 53, 54 in such a way that said members lie in planes different from each other. The mounting supports 2; 21, 22 are attached to the frame 5, most preferably by the ends thereof, The mounting supports 2; 21, 22 are attached to the opposite parallel frame members 51, 52; 53, 54 of the frame 5.

In one preferred embodiment of the invention, the mounting supports 2; 21, 22 are attached movably to the frame 5; 51, 52 in such a way that individual mounting supports in each group 21, 22 are movable relative to each other in the direction of the mounting level A-A or B-B, respectively, such that the actuation relative to each other of the mounting supports included in a specific group 21 or 22 maintains the air resistance established by the lighthead 1 unchanged in the direction C-C transverse to the mounting levels A-A and B-B. Thus, the gaps 4; 4a, 4b between the mounting supports are also changeable, but most preferably in such a way that there will be at least a small gap, a minimum gap, between the mounting supports.

The airflow, which passes through the light head 1, arrives from the incoming air frame TF and proceeds through the lighthead's mounting supports included in the groups 21 and 22 in the direction C-C transverse, preferably perpendicular, to the respective mounting levels A-A and B-B. Thus, the fact that the lighthead's air resistance remains unchanged results at the same time in a consequence that, as the lighthead 1 is placed below the incoming air frame TF and at the same time above the operating table LP, the laminar airflow arriving from the incoming air frame TF penetrates the lighthead 1 in the direction C-C while retaining its laminarity.

Actuation of the mounting supports 2 can be effected for example in such a way that each mounting support 21; 21¹, 21²; 22; 22¹, 22² has its opposite ends accommodated on guides included in the frame 5, such as in the opposite frame members 51, 52; 53, 54, along which guides the mounting support is movable with suitable transfer means. Hence, the mounting supports 2 are then actuated along the guides preferably in such a way that the direction of each mounting support's vertical axis, i.e. the direction of that particular axis which extends in a direction transverse to the direction of the mounting levels A-A and B-B, shall be maintained constant relative to the average direction of an airflow arriving at the lighthead in the direction C-C.

Said guides may be constituted by the frame members themselves. These transfer means are implementable e.g. by means of a toothed rack or belt fitted on the guide and a gear disposed on the end of a mounting support, and by means of an electric motor, such as a stepped motor, driving the same. It should be appreciated, however, that there are a wide variety of possibilities of realizing the transfer means

The frame 5, especially the frame members 51, 52, 53, 54, is most preferably constructed from long rod type elements, which in one embodiment of the invention are essentially similar to the mounting supports 21; 21¹, 21²; 22; 22¹, 22². It should be noted, however, that other sort of long rod type or shaft type members can also be used in the frame.

The lighthead 1 is suspended by various sides thereof with suitable suspension elements, such a plurality of suspension ropes 62 or the like, from ceiling structures, especially outside an area defined by the incoming air frame TF of an operating theatre.

A preferred embodiment for a lighting arrangement of the invention comprises also displacement elements 6, which enable the lighthead 1 to be raised or lowered as necessary. The displacement elements 6 comprise a power unit such as one or more electric motors 61, and suspension elements such as a plurality of suspension ropes 62 or the like between the frame 5 and the drive shaft of a power unit such as the electric motor 61. In the embodiment shown in the drawing, the number of suspension elements, such as the suspension ropes 62, and the number of electric motors 61 is four on various sides of the frame 5 in the proximity of the corners of the rectangular-shaped lighthead 1 at ceiling structures. The displacement elements 6 enable a manipulation of the lighthead 1, i.e. the frame 5 and the mounting supports 2, in vertical direction. The lighthead 1 can thus be lowered downward from ceiling structures to a desired level and respectively raised back upward to the proximity of ceiling structures. The lighthead 1 can also be set in an inclined position by means of the displacement elements 6, if that is deemed necessary.

A preferred embodiment for a lighting arrangement of the invention comprises at least one accessory 7, such as a digital camera 71 and/or an electronic pointing device (a touchpad) 72 or a generally applicable remote controller.

The camera 71 is fitted in connection with the lighthead 1, such as a suitable light support 2, and it is pointed to image in the illumination direction of the light elements 3 at least a part of the area to be illuminated. The camera 71 is used for monitoring the area to be illuminated. A screen 72a of the electronic pointing device 72 is used for viewing an image given by the camera 71 and for determining, e.g. by touching or a cursor movement, a part of the camera coverage area desired for concentrating the illumination and for focusing the light field. After this, a lighting arrangement control unit 8 (described hereinafter), with a suitable control program stored therein, issues control commands for at least some of the light elements 3 for concentrating their light beams upon an appropriate area, such as upon the body part of a patient resting on the operating table intended for a surgical procedure. The electronic point device 72 is also preferably used for determining the size of an area intended for powerful illumination, and a desired light intensity/light flux implemented by means of the control unit 8. The camera 71 is used for monitoring the obtained light area and light intensity ad for keeping the same essentially within definitions. Alternatively, the monitoring of a light intensity received by an object is conducted with a suitable optical sensor on the basis of light reflected from the object.

Whenever necessary, the mounting supports 2; 21, 22 can be displaced and/or the light elements 3 can be repositioned in the mounting supports and/or the frame can be raised/lowered for ensuring that the desired area is provided with best possible illumination in terms of both the light field shape and the light intensity. All these actions are carried out by means of appropriate control programs stored in the control unit 8.

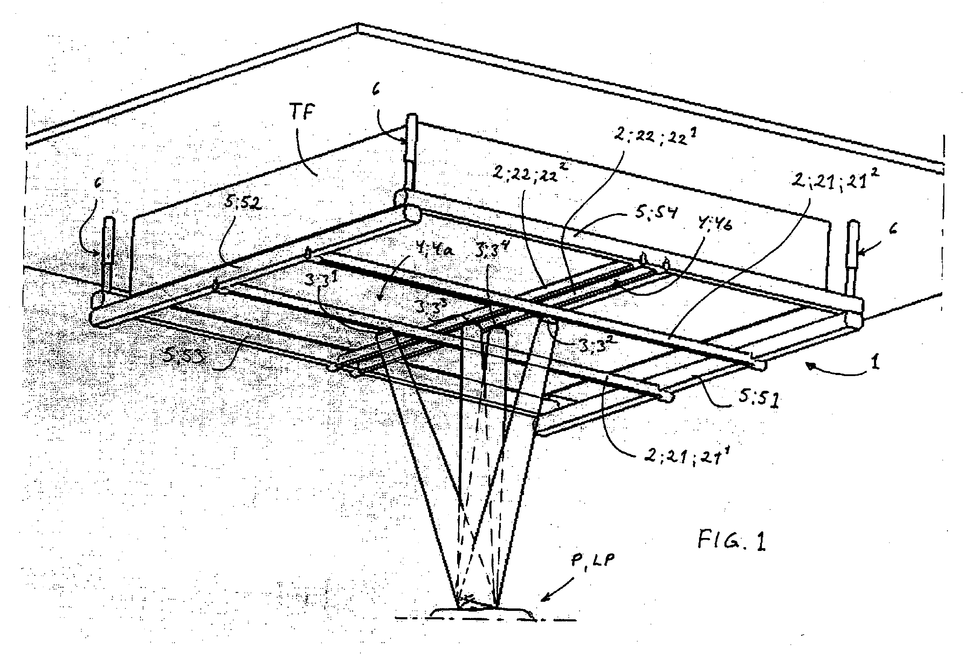

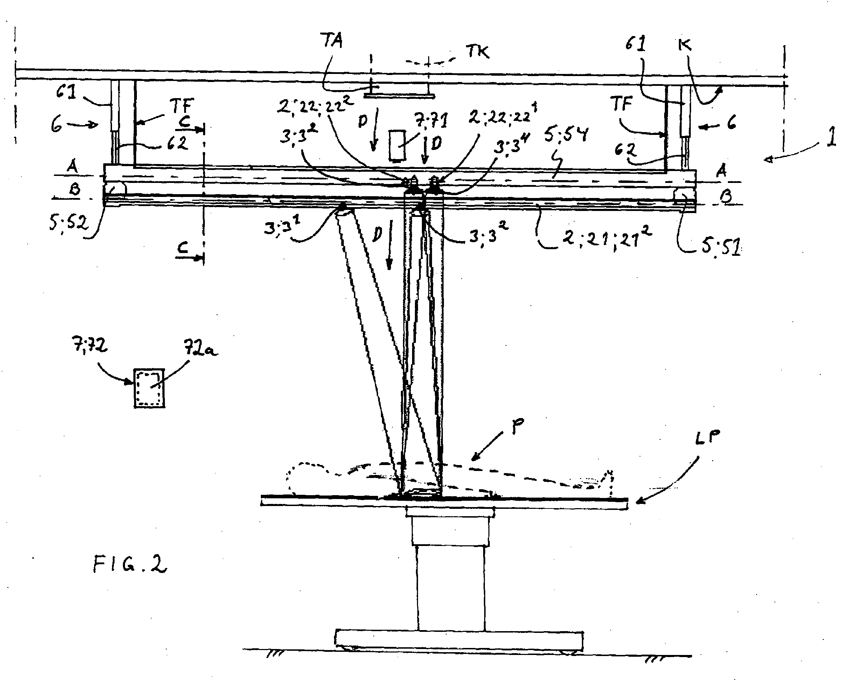

The lighting arrangement according to the invention lends itself particularly well to an operating theatre or the like facility, such as a clean room. The lighthead 1, along with its mounting supports 2; 21, 22; 21¹, 21², 22¹, 22² and its frame 5, is disposed in the proximity of the operating theatre ceiling K above the operating table LP, as specifically illustrated in fig. 1. Especially in an operating theatre, the lighthead 1 is installed within an area defined by the incoming air frame TF and directly below this area. Consequently, the lighthead 1 is located below one or more air inlets TA of an incoming air duct TK, whereby the incoming air (arrows D in fig. 2) is able to discharge in an essentially laminar flow from the air inlets inside the incoming air frame through the lighthead 1, in other words through the gaps 4; 4a, 4b between the mounting supports 2; 21, 22; 21¹, 21²; 22¹, 22² of the lighthead 1, down towards the operating table LP and a patient P presently thereon, and to disperse away from the operating table into outgoing air ducts or the like air outlet channels.

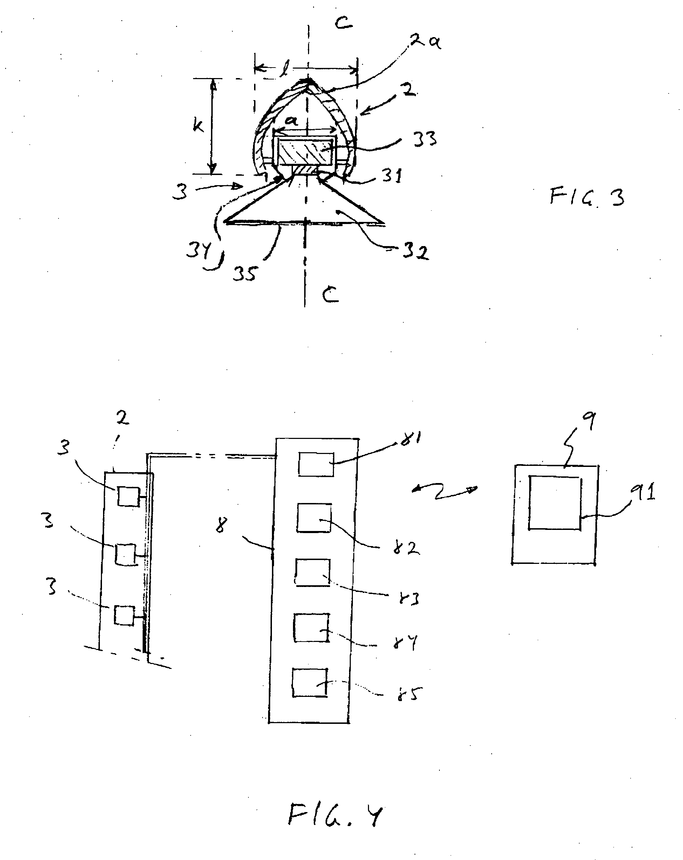

In a preferred embodiment of the invention, the lighting arrangement further comprises the control unit 8, fig. 4, for adjusting at least the light elements 3 disposed on the light supports 2 of the lighthead 1. The control unit 8 comprises preferably at least three adjustment units 81, 82, 83.

The first adjustment unit 81 is intended for managing the power supply to the light source 31 of each light element 2 and/or for adjusting the light intensity. The first adjustment unit 81 is connected to the light source 31 of each light element 3, particularly to its power supply V, for allowing and disrupting the power supply by way of the first adjustment unit 81 to the discussed light source, as well as for adjusting this power and thereby the light intensity within a predefined range. In case the light element 3 contains a number of LEDs, the spectral ranges of light produced thereby being different from each other, it is also possible to adjust the color temperature of light produced by the light element 3.

The second adjustment unit 82 is intended for adjusting the optics 32 of each light element 3 and for concentrating a light beam 311 produced by the light element 3 at a desired distance. Hence, this enables the light beam 311 of each light element 3 to be focused e.g. on a specific level as regarded a.o. from the plane of an operating table. The second adjustment unit 82 is connected to an adjustment element for the optics 32 of each light element 3.

The third adjustment unit 83 is intended for controlling alignment elements 33 for the light source 31 of each light element 3, such as one or more electric motors and a leverage or the like linked to the light element. By means of the alignment elements, the light element 3 and at the same time the light beam 311 produced thereby can be tilted 10-15 degrees, most preferably in any direction, from an axis perpendicular to the level A-A (or B-B). Therefore, the light beam 311 produced by each light element 3 can be directed onto a predefined part of the operating table and particularly the surgical patient.

The third adjustment unit 83 is used for controlling most preferably also a possible actuation of the light elements 3 in the mounting supports 2.

The control unit 8 comprises preferably also a fourth adjustment unit 84, which is intended for controlling displacement elements 6, 7, such as one or more electric motors 61, for raising or lowering the frame 5. By means of the control unit 8, particularly by means of the fourth adjustment unit 84, each electric motor is controlled so as to enable the attainment of a desired level for the lighthead 1 as determined e.g. from the floor level.

The fourth control unit 84 is used for controlling most preferably also a possible shifting of the mounting supports 2; 21, 22 in the frame 5.

The control unit 8 further comprises most preferably a plurality of sensors, which are connected to various adjustment units 81, 82, 83, 84 for obtaining measurement data about the lighting arrangement for carrying out and monitoring the adjustment functions.

An accessory 7, such as the digital camera 71 and/or the electronic pointing device (a touchpad) 72, is connected to the control unit 8. The information obtained from the accessory 7; 71, 72 is utilized in the control unit 8 and, respectively, the accessories are operated and adjusted by means of the control unit.

The control unit 8 further comprises one or more monitoring units 85, which are used for monitoring e.g. safety of the lighting arrangement and for reporting possible malfunctions.

The control unit 8, especially its adjustment and monitoring units 81, 82, 83, 84, 85, is used for monitoring and adjusting at least one of the following features of the light element 3: switching the light element on/off and the order of magnitude of its light intensity (and color temperature), setting the focus of a light beam and the direction of a light beam. Hence, at least some of the light produced by the light elements 3 can be focused on a patient on the operating table and, in addi6tion, on a desired (target) area.

The control unit 8, along with its adjustment and monitoring units 81, 82, 83, 84, 85, is most preferably implemented by means of a microprocessor or the like data processing unit. In this case, the control unit 8 has its functions executed by means of computer programs and, thus, the lighting arrangement operates in a program-controlled fashion.

In a preferred embodiment of the invention, the lighting arrangement comprises a remote controller 9. This is most preferably in a wireless (radio or infrared link) communication with the control unit 8, especially with its adjustment and monitoring units 81, 82, 83, 84, 85. This way, the remote controller is used for controlling and adjusting the lighting arrangement and its various functions. The remote controller 9 is most preferably provided with a touch screen 91. Various symbols for functions of the lighting arrangement and adjustable features of the light elements 2 are exhibitable on the touch screen.

The invention is not limited concern just the foregoing exemplary embodiment, but many modifications are possible while remaining within the scope of the inventive concept defined in the claims.

## Claims

1. An improved lighting arrangement, which is implemented by means of light emitting diodes or LEDs, wherein the lighting arrangement comprises a lighthead (1) and an incoming air frame (TF), which lighthead (1) is constituted by a frame (5; 51, 52, 53, 54) fitted with a plurality of elongated rod type mounting supports (2; 21, 22), said mounting supports (2; 21; 22) being provided with a plurality of light elements (3; 3¹, 3², 3³) containing at least one light emitting diode (31), whereby the mounting supports (2) are organized in at least two groups (21, 22), each of said groups (21, 22) including at least two mounting supports (21; 21¹, 21²; 22; 22¹, 22²), whereof the first group (21) is set on a first level (A-A) and the second group on a second level (B-B), such that the mounting supports (21; 21¹, 21²; 22; 22¹, 22²) of each group (21, 22) are apart or spaced by a gap (4; 4a, 4b) from each other and that the first group's mounting supports (21) lie at an angle, most preferably at a right angle, relative to the second group's (22) mounting supports, and the lighthead (1), along with its frame (5) and mounting supports (2; 21, 22), is set up in the proximity of a ceiling (K) of the operating theatre or the like facility substantially above an operating table (LP), such that the location thereof is below the illuminated facility's incoming air frame (TF).

2. A lighting arrangement as set forth in claim 1, **characterized in that** the first and second groups' mounting supports (21; 21¹, 21², 22; 22¹, 22²), and at the same time also the levels (A-A and B-B), on which the mounting supports (21; 21¹, 21², 22; 22¹, 22²) of said groups are set, are located at a small distance, such as 25-50 mm, from each other.

3. A lighting arrangement as set forth in claim 1 or 2, **characterized in that** the mounting supports (2) in each group (21, 22) extend in a mutually parallel relationship at a specific distance (4a, 4b) from each other.

4. A lighting arrangement as set forth in any of the preceding claims, **characterized in that** both the first group's mounting supports (21; 21¹, 21²) and also the second group's mounting supports (22; 22¹, 22²) exhibit a certain low air resistance in a direction (C-C) transverse, preferably in a direction perpendicular, relative to the mounting levels (A-A ad B-B) established by the mounting supports (2) of said groups (21, 22).

5. A lighting arrangement as set forth in claim 4, **characterized in that** the first and second groups' mounting supports (2) are aerodynamic rod type or shaft type elements, the air arriving at said mounting supports from the incoming air frame (TF) being able to flow through the gaps (4; 4a, 4b) therebetween.

6. A lighting arrangement as set forth in any of the preceding claims, **characterized in that** the laminar airflow arriving at the incoming air frame (TF) passes through the lighthead (1) while essentially retaining its laminarity.

7. A lighting arrangement as set forth in any of the preceding claims, **characterized in that** the mounting supports (2) are attached to the frame (5) in a movable manner, such that the individual mounting supports of each group (21, 22) are movable relative to each other.

8. A lighting arrangement as set forth in claim 7, **characterized in that** the frame (5; 51, 52, 53, 54) is provided with guides, on which are adapted the ends of each mounting support (21; 21¹, 21²; 22; 22¹, 22²), the mounting support being movable along said guides with suitable transfer means.

9. A lighting arrangement as set forth in claim 8, **characterized in that** the mounting supports (2; 21, 22) are movable along the guides in such a way that the air resistance thereof remains constant in the direction (C-C) transverse, preferably in a direction perpendicular, relative to the mounting levels (A-A and B-B) established by the mounting supports (2) of the groups (21, 22).

10. A lighting arrangement as set forth in claim 9, **characterized in that** the mounting supports (2) are moved along the guides in such a way that the lighthead's surface area, which is penetrated by the airflow arriving at the lighthead (1) from the incoming air frame (TF) in the direction (C-C) transverse with respect to the mounting levels (A-A and B-B), remains essentially unchanged in the direction of the mounting levels (A-A and B-B).

11. A lighting arrangement as set forth in claim 9 or 10, **characterized in that** the mounting supports (2) are moved along the guides in such a way that the mounting support's orientation in the direction (C-C) transverse relative to the mounting levels (A-A and B-B) remains constant with respect to the airflow arriving at the lighthead from the incoming air frame (TF).

12. A lighting arrangement as set forth in any of the preceding claims, **characterized in that** the light elements (3; 3¹, 3², 3³) are fitted on the mounting support (2; 21; 21¹, 21²; 22; 22¹, 22²) in a row successively at a distance from each other.

13. A lighting arrangement as set forth in any of the preceding claims, **characterized in that** at least some of the light elements (3; 3¹, 3², 3³) comprise not only a light source (31), i.e. a LED, but also adjustable optics (32) for focusing the light, and alignment elements, such as one or more reversible motors (33), for directing the light, especially a light beam (311), emitted by the light source.

14. A lighting arrangement as set forth in claim 5 or 6, **characterized in that** at least some of the light elements (3; 3¹, 3², 3³) are adapted to be movable in the mounting support (2; 21, 22) in its longitudinal direction.

15. A lighting arrangement as set forth in any of the preceding claims, **characterized in that** the lighting arrangement comprises displacement elements (6; 61, 62), by means of which the lighthead (1) is capable of being raised and/or lowered as necessary.

16. A lighting arrangement as set forth in any of the preceding claims, **characterized in that** the lighting arrangement further comprises a control unit (8) for adjusting at least one of the following features of the light elements (3; 3¹, 3², 3³): switching the light element on/off and the order of magnitude of its light intensity, setting the focus of a light beam and the direction of a light beam, the position of a light element on the mounting support, the position of a mounting support with respect to the frame.

17. A lighting arrangement as set forth in claim 16, **characterized in that** the lighting arrangement comprises a remote controller (9) for adjusting the lighting arrangement through the intermediary of the control unit (8).

18. A lighting arrangement as set forth in claim 10, **characterized in that** the lighting arrangement comprises at least one accessory (7), such as a digital camera (71) and an electronic pointing device (72).

## Patentansprüche

1. Verbesserte Beleuchtungsanordnung, die mithilfe von Leuchtdioden oder LEDs realisiert ist, wobei die Beleuchtungsanordnung einen Leuchtkopf (1) und einen Zuluftrahmen (TF) umfasst, wobei der Leuchtkopf (1) durch einen Rahmen (5; 51, 52, 53, 54) gebildet ist, der mit mehreren lang gestreckten stabartigen Halterungen (2; 21, 22) ausgestattet ist, wobei die Halterungen (2; 21, 22) mit mehreren Leuchtelementen (3; 3¹, 3², 3³) versehen sind, die mindestens eine Leuchtdiode (31) enthalten, wobei die Halterungen (2) in mindestens zwei Gruppen (21, 22) organisiert sind, wobei jede der Gruppen (21, 22) mindestens zwei Halterungen (21; 21¹, 21²; 22; 22¹, 22²) aufweist, von denen die erste Gruppe (21) auf eine erste Ebene (A-A) und die zweite Gruppe auf eine zweite Ebene (B-B) gesetzt ist, derart dass die Halterungen (21; 21¹, 21²; 22; 22¹, 22²) jeder Gruppe (21, 22) durch einen Spalt (4; 4a, 4b) voneinander entfernt oder beabstandet sind und dass die Halterungen (21) der ersten Gruppe in einem Winkel, ganz besonders bevorzugt in einem rechten Winkel, zu den Halterungen der zweiten Gruppe (22) liegen und der Leuchtkopf (1), zusammen mit seinem Rahmen (5) und seinen Halterungen (2; 21, 22) in der Nähe einer Decke (K) des Operationssaals oder der ähnlichen Einrichtung, im Wesentlichen über einem Operationstisch (LP), angeordnet ist, derart, dass die Position desselben unter dem Zuluftrahmen (TF) der beleuchteten Einrichtung ist.

2. Beleuchtungsanordnung nach Anspruch 1, **dadurch gekennzeichnet, dass** die Halterungen (21; 21¹, 21²; 22; 22¹, 22²) der ersten und zweiten Gruppe und gleichzeitig auch die Ebenen (A-A und B-B), auf welche die Halterungen (21; 21¹, 21²; 22; 22¹, 22²) der Gruppen gesetzt sind, sich in einem kleinen Abstand, wie z. B. 25 bis 50 mm, voneinander befinden.

3. Beleuchtungsanordnung nach Anspruch 1 oder 2, **dadurch gekennzeichnet, dass** sich die Halterungen (2) in jeder Gruppe (21, 22) in einem zueinander parallelen Verhältnis in einem bestimmten Abstand (4a, 4b) voneinander erstrecken.

4. Beleuchtungsanordnung nach einem der vorhergehenden Ansprüche, **dadurch gekennzeichnet, dass** sowohl die Halterungen (21; 21¹, 21²) der ersten Gruppe als auch die Halterungen (22; 22¹, 22²) der zweiten Gruppe in einer Richtung (C-C), quer, vorzugsweise in einer Richtung senkrecht, zu den Befestigungsebenen (A-A und B-B), die durch die Halterungen (2) der Gruppen (21, 22) begründet werden, einen gewissen niedrigen Luftwiderstand aufweisen.

5. Beleuchtungsanordnung nach Anspruch 4, **dadurch gekennzeichnet, dass** die Halterungen (2) der ersten und zweiten Gruppe aerodynamische stabartige oder stangenartige Elemente sind, wobei die Luft, die von dem Zuluftrahmen (TF) her an den Halterungen ankommt, in der Lage ist, durch die Spalte (4; 4a, 4b) zwischen denselben zu strömen.

6. Beleuchtungsanordnung nach einem der vorhergehenden Ansprüche, **dadurch gekennzeichnet, dass** die laminare Luftströmung, die an dem Zuluftrahmen (TF) ankommt, den Leuchtkopf (1) passiert, während sie ihre Laminarität im Wesentlichen behält.

7. Beleuchtungsanordnung nach einem der vorhergehenden Ansprüche, **dadurch gekennzeichnet, dass** die Halterungen (2) in einer beweglichen Weise an dem Rahmen (5) angebracht sind, derart dass die einzelnen Halterungen jeder Gruppe (21, 22) bezogen aufeinander beweglich sind.

8. Beleuchtungsanordnung nach Anspruch 7, **dadurch gekennzeichnet, dass** der Rahmen (5; 51, 52, 53, 54) mit Führungen versehen ist, auf denen die Enden jeder Halterung (21; 21¹, 21²; 22; 22¹, 22²) angepasst sind, wobei die Halterung mit geeigneten Transportmitteln entlang der Führungen beweglich ist.

9. Beleuchtungsanordnung nach Anspruch 8, **dadurch gekennzeichnet, dass** die Halterungen (2; 21, 22) entlang der Führungen derart beweglich sind, dass der Luftwiderstand derselben in der Richtung (C-C), quer, vorzugsweise in einer Richtung senkrecht, zu den Befestigungsebenen (A-A und B-B), die durch die Halterungen (2) der Gruppen (21, 22) begründet werden, konstant bleibt.

10. Beleuchtungsanordnung nach Anspruch 9, **dadurch gekennzeichnet, dass** die Halterungen (2) entlang der Führungen derart bewegt werden, dass die Oberfläche des Leuchtkopfes, die von dem Luftstrom, der von dem Zuluftrahmen (TF) her an dem Leuchtkopf (1) ankommt, in der Richtung (C-C), quer zu den Befestigungsebenen (A-A und B-B), durchdrungen wird, in der Richtung der Befestigungsebenen (A-A und B-B) im Wesentlichen unverändert bleibt.

11. Beleuchtungsanordnung nach Anspruch 9 oder 10, **dadurch gekennzeichnet, dass** die Halterungen (2) entlang der Führungen derart bewegt werden, dass die Orientierung der Halterung in der Richtung (C-C), quer zu den Befestigungsebenen (A-A und B-B), bezogen auf den Luftstrom, der von dem Zuluftrahmen (TF) her an dem Leuchtkopf ankommt, konstant bleibt.

12. Beleuchtungsanordnung nach einem der vorhergehenden Ansprüche, **dadurch gekennzeichnet, dass** die Leuchtelemente (3; 3¹, 3², 3³) auf der Halterung (2; 21; 21¹, 21²; 22; 22¹, 22²) in einer Reihe, in einem Abstand voneinander aufeinander folgend, angebracht sind.

13. Beleuchtungsanordnung nach einem der vorhergehenden Ansprüche, **dadurch gekennzeichnet, dass** mindestens einige der Leuchtelemente (3; 3¹, 3², 3³) nicht nur eine Lichtquelle (31), d. h. eine LED, sondern auch eine einstellbare Optik (32) zum Fokussieren des Lichts und Ausrichtungselemente, wie z. B. einen oder mehrere Umkehrmotor(en) (33), zum Richten des Lichts, insbesondere eines Lichtstrahls (311), der durch die Lichtquelle ausgesandt wird, umfassen.

14. Beleuchtungsanordnung nach Anspruch 5 oder 6, **dadurch gekennzeichnet, dass** mindestens einige der Leuchtelemente (3; 3¹, 3², 3³) dazu ausgelegt sind, in der Halterung (2; 21, 22) in ihrer Längsrichtung beweglich zu sein.

15. Beleuchtungsanordnung nach einem der vorhergehenden Ansprüche, **dadurch gekennzeichnet, dass** die Beleuchtungsanordnung Verlagerungselemente (6; 61, 62) umfasst, mittels derer der Leuchtkopf (1) nach Bedarf angehoben und/oder abgesenkt werden kann.

16. Beleuchtungsanordnung nach einem der vorhergehenden Ansprüche, **dadurch gekennzeichnet, dass** die Beleuchtungsanordnung ferner eine Steuereinheit (8) zum Einstellen mindestens eines der folgenden Merkmale der Leuchtelemente (3; 3¹, 3², 3³) umfasst: Ein/Ausschalten des Leuchtelements und Umschalten der Größenordnung seiner Lichtstärke, Einstellen des Fokus eines Lichtstrahls und der Richtung eines Lichtstrahls, der Position eines Leuchtelements auf der Halterung, der Position einer Halterung mit Bezug auf den Rahmen.

17. Beleuchtungsanordnung nach Anspruch 16, **dadurch gekennzeichnet, dass** die Beleuchtungsanordnung eine Fernsteuerung (9) zum Einstellen der Beleuchtungsanordnung über die Steuereinheit (8) umfasst.

18. Beleuchtungsanordnung nach Anspruch 10, **dadurch gekennzeichnet, dass** die Beleuchtungsanordnung mindestens ein Zubehörteil (7), wie z. B. eine Digitalkamera (71) und eine elektronische Zeigevorrichtung (72), umfasst.

## Revendications

1. Agencement d'éclairage amélioré, qui est mis en oeuvre au moyen de diodes électroluminescentes, LED, dans lequel l'agencement d'éclairage comprend une tête d'éclairage (1) et un cadre d'air entrant (TF), ladite tête d'éclairage (1) est constituée d'un cadre (5 ; 51, 52, 53, 54) pourvu d'une pluralité de supports de montage de type à tige allongée (2 ; 21, 22), lesdits supports de montage (2 ; 21, 22) étant pourvus d'une pluralité d'éléments d'éclairage (3 ; 3¹, 3², 3³) contenant au moins une diode électroluminescente (31), de telle manière que les supports de montage (2) soient organisés en au moins deux groupes (21, 22), chacun desdits groupes (21, 22) comprenant au moins deux supports de montage (21 ; 21¹, 21² ; 22 ; 22¹, 22²), parmi lesquels le premier groupe (21) est placé à un premier niveau (A-A) et le deuxième groupe est placé à un deuxième niveau (B-B), de sorte que les supports de montage (21 ; 21¹, 21² ; 22 ; 22¹, 22²) de chaque groupe (21, 22) soient séparés ou espacés d'un espacement (4 ; 4a, 4b) l'un de l'autre et que les supports de montage du premier groupe (21) se trouvent à un angle, avec le plus de préférence à un angle droit, par rapport aux supports de montage du deuxième groupe (22), et la tête d'éclairage (1), le long de son cadre (5) et des supports de montage (2 ; 21, 22), soit configurée à proximité d'un plafond (K) de la salle d'opération ou d'une installation similaire sensiblement au-dessus d'une table d'opération (LP), afin que son emplacement soit au-dessous du cadre d'air entrant (TF) de l'installation éclairée.

2. Agencement d'éclairage selon la revendication 1, **caractérisé en ce que** les supports de montage (21 ; 21¹, 21² ; 22 ; 22¹, 22²) du premier groupe et du deuxième groupe et, en même temps, les niveaux (A-A et B-B) sur lesquels les supports de montage (21 ; 21¹, 21² ; 22 ; 22¹, 22²) desdits groupes sont placés se trouvent à une petite distance, comme de 25 à 50 mm, l'un de l'autre.

3. Agencement d'éclairage selon la revendication 1 ou 2, **caractérisé en ce que** les supports de montage (2) dans chaque groupe (21, 22) s'étendent dans une relation mutuellement parallèle à une distance spécifique (4a, 4b) l'un de l'autre.

4. Agencement d'éclairage selon l'une quelconque des revendications précédentes, **caractérisé en ce que** les supports de montage (21 ; 21¹, 21²) du premier groupe et les supports de montage (22 ; 22¹, 22²) du deuxième groupe présentent une certaine faible résistance à l'air dans un sens (C-C) transversal, de préférence dans un sens perpendiculaire, par rapport aux niveaux de montage (A-A et B-B) établis par les supports de montage (2) desdits groupes (21, 22).

5. Agencement d'éclairage selon la revendication 4, **caractérisé en ce que** les supports de montage des premier et deuxième groupes (2) sont des éléments de type à tige aérodynamique ou de type à arbre, l'air arrivant aux dits supports de montage depuis le cadre d'air entrant (TF) pouvant s'écouler à travers les espacements (4 ; 4a, 4b) entre ceux-ci.

6. Agencement d'éclairage selon l'une quelconque des revendications précédentes, **caractérisé en ce que** le flux d'air laminaire arrivant au cadre d'air d'entrée (TF) passe à travers la tête d'éclairage (1) en conservant sensiblement sa laminarité.

7. Agencement d'éclairage selon l'une quelconque des revendications précédentes, **caractérisé en ce que** les supports de montage (2) sont attachés au cadre (5) de manière à pouvoir se déplacer, afin que les supports de montage individuels de chaque groupe (21, 22) puissent se déplacer l'un par rapport à l'autre.

8. Agencement d'éclairage selon la revendication 7, **caractérisé en ce que** le cadre (5 ; 51, 52, 53, 54) est pourvu de guides sur lesquels sont adaptées les extrémités de chaque support de montage (21 ; 21¹, 21² ; 22, 22¹, 22²), le support de montage pouvant se déplacer le long desdits guides avec des moyens de transfert appropriés.

9. Agencement d'éclairage selon la revendication 8, **caractérisé en ce que** les supports de montage (2 ; 21, 22) peuvent se déplacer le long des guides de telle manière que leur résistance à l'air reste constante dans le sens (C-C) transversal, de préférence dans un sens perpendiculaire, par rapport aux niveaux de montage (A-A et B-B) établis par les supports de montage (2) des groupes (21, 22).

10. Agencement d'éclairage selon la revendication 9, **caractérisé en ce que** les supports de montage (2) sont déplacés le long des guides de telle manière que l'aire de surface de la tête d'éclairage, qui est pénétrée par le flux d'air arrivant à la tête d'éclairage (1) depuis le cadre d'air entrant (TF) dans le sens (C-C) transversal par rapport aux niveaux de montage (A-A et B-B) reste sensiblement inchangée dans le sens des niveaux de montage (A-A et B-B).

11. Agencement d'éclairage selon la revendication 9 ou 10, **caractérisé en ce que** les supports de montage (2) sont déplacés le long des guides de telle manière que l'orientation des supports de montage dans le sens (C-C) transversal par rapport aux niveaux de montage (A-A et B-B) reste constante par rapport au flux d'air arrivant à la tête d'éclairage depuis le cadre d'air entrant (TF).

12. Agencement d'éclairage selon l'une quelconque des revendications précédentes, **caractérisé en ce que** les éléments d'éclairage (3 ; 3¹, 3², 3³) sont montés sur le support de montage (2 ; 21 ; 21¹, 21² ; 22 ; 22¹, 22²) dans une rangée successivement à une distance l'un de l'autre.

13. Agencement d'éclairage selon l'une quelconque des revendications précédentes, **caractérisé en ce qu'**au moins certains des éléments d'éclairage (3 ; 3¹, 3², 3³) comprennent non seulement une source lumineuse (31), c'est-à-dire une LED, mais également un dispositif optique ajustable (32) pour focaliser la lumière, et des éléments d'alignement, comme un ou plusieurs moteurs réversibles (33), pour diriger la lumière, particulièrement un faisceau lumineux (311), émise par la source lumineuse.

14. Agencement d'éclairage selon la revendication 5 ou 6, **caractérisé en ce qu'**au moins certains des éléments d'éclairage (3 ; 3¹, 3², 3³) sont aptes à pouvoir se déplacer dans le support de montage (2 ; 21, 22) dans son sens longitudinal.

15. Agencement d'éclairage selon l'une quelconque des revendications précédentes, **caractérisé en ce que** l'agencement d'éclairage comprend des éléments de déplacement (6 ; 61, 62) au moyen desquels la tête d'éclairage (1) est capable d'être soulevée et/ou abaissée selon les besoins.

16. Agencement d'éclairage selon l'une quelconque des revendications précédentes, **caractérisé en ce que** l'agencement d'éclairage comprend en outre une unité de commande (8) pour ajuster au moins l'une des caractéristiques suivantes des éléments d'éclairage (3 ; 3¹, 3², 3³) : la mise sous tension/hors tension de l'élément d'éclairage et l'ordre de grandeur de son intensité lumineuse, le réglage de la focalisation d'un faisceau lumineux et le sens d'un faisceau lumineux, la position d'un élément d'éclairage sur le support de montage, la position d'un support de montage par rapport au cadre.

17. Agencement d'éclairage selon la revendication 16, **caractérisé en ce que** l'agencement d'éclairage comprend une télécommande (9) pour ajuster l'agencement d'éclairage par l'intermédiaire de l'unité de commande (8).

18. Agencement d'éclairage selon la revendication 10, **caractérisé en ce que** l'agencement d'éclairage comprend au moins un accessoire (7), comme une caméra numérique (71) et un dispositif de pointage électronique (72).
